# EUROPEAN PATENT APPLICATION

(11) **EP 2 668 915 A1**
(43) Date of publication of application: **04.12.2013**
(21) Application number: 12181594.8
(22) Date of filing: 23.08.2012
(51) Int. Cl.: A61B 17/12

(54) **System for delivering a stretch resistant vaso-occlusive device and a method of producing same**

(30) Priority: 01.06.2012 EP 12170491
(71) Applicant: Acandis GmbH & Co. KG, 76327 Pfinztal (DE)
(72) Inventor: Watson, David, San Jose, CA California 95125 (US)
(74) Representative: Kilchert, Jochen

(57) **Abstract**

Provided is a system for delivering a vaso-occlusive device for release within the vasculature of a patient wherein the vaso-occlusive device (10) comprises an outer helically wound primary coil (14) having a proximal end (16) and a distal end (17), defining a lumen (15) between said proximal end and distal end. The proximal end of the vaso-occlusive device is detachably mounted to the distal end (33) of an elongated delivery means (30). Further, the system also comprises an attachment element (19), in particular a first attachment element, at the distal end of the vaso-occlusive device and a stretch resisting member (20) having at least two proximal ends and at least one distal end and extending at least partially through said lumen and passing through said attachment element wherein said distal end of stretch resisting member is releasably attached to said distal attachment element. Thus, the present invention has the advantage that the vaso-occlusive device is not damaged during manipulation by a physician and thus ensures that the risk of injury to a subject is reduced during a medical procedure. The present invention also provides a method for producing a system for delivering a vaso-occlusive device within the vasculature of a patient.

## Description

### Field of the Invention

The invention relates to the field of vaso-occlusive devices for the treatment of various vascular anomalies such as for example aneurysms, arterio-venous fistula, and artery occlusions. More particularly, the invention provides a means for delivering a stretch resistant vaso-occlusive device within a vasculature of a patient that after deployment conforms to the shape of an anatomical cavity. Further, the invention relates to a method of producing a system for delivering a vaso-occlusive.

### Background of the Invention

The treatment of vascular anomalies such as aneurysms, arterio-venous fistula, and artery occlusions or other vascular anomalies remains an important area of research both to physicians and patients alike and has improved dramatically in recent years by placement of devices within a vasculature of a patient such that many vascular anomalies are treated in-situ.

In general, vaso-occlusion devices are therapeutic devices that are placed within the vasculature of the human body, typically via a catheter, either to block the flow of blood through a vessel making up that portion of the vasculature through the formation of an embolus or to form such an embolus within an aneurysm stemming from the vessel, thus ultimately healing the aneurysm.

Moreover, such devices allow a physician to treat a patient minimally invasively so that exposure to risk factors associated with invasive surgery is minimized. That is, the placement of vaso-occlusive devices within a vasculature of a patient allows the physician to treat a patient without using surgical techniques that are considered higher risk to the patient.

These vaso-occlusive devices can be produced in such a way that they will pass through the lumen of a catheter in a linear shape and take on a complex shape as originally formed after being deployed into the area of interest, such as an aneurysm. These devices are commonly produced as primary coiled winding of an implantable metal that is subsequently formed into a more complex secondary shape by winding the primary winding about poles placed on a winding mandrel. The secondary wound coil is then annealed on the winding mandrel, and the coil is then removed from the winding mandrel. One widely used vaso-occlusive device as described in US patent 5,645,558 is a helical wire coil having a deployed configuration which is generally spherical shaped and can be dimensioned to engage the walls of the vessels or aneurysm. The delivery of such vaso-occlusive devices can be accomplished by a variety of means, including via a catheter in which the device is pushed through the catheter by a pusher to deploy the device. The vaso-occlusive device is releasably attached to a pusher member such that the physician may manipulate its position prior to detaching it in-situ. Various detachment mechanisms to release the device from a pusher have been developed and are known in the art.

For treatment of areas of the small diameter vasculature such as a small artery or vein in the brain, for example, and for treatment of aneurysms and the like, the vaso-occlusive device may take the form of a micro-coil. These micro-coils are formed from very small diameter wire are used in order to restrict, reinforce, or to occlude such small diameter areas of the vasculature. The primary winding of these micro-coils is generally formed from a very small metallic wire with a diameter generally ranging from 40 to 80 micrometers. This wire is formed into a primary coil generally about 0.25mm - 0.50mm in diameter such that it can be inserted in a linear configuration up a correspondingly sized microcatheter. A variety of materials have been suggested for use in such micro-coils, including nickel-titanium alloys, copper, stainless steel, platinum, tungsten, various plastics or the like, each of which offers certain benefits in various applications. Platinum alloys are particularly advantageous for the fabrication of such micro coils, in that they can have suitable shape memory properties, radiopacity to be seen with x-ray during deployment, and can be manufactured to easily fit into a linear portion of a catheter, yet revert to their originally formed, more complex shape when deployed.

One described vaso-occlusive coil is known from US 6,638,291, for example, that has a three dimensional in-filling coil configuration, formed by winding a wire into a primary helix, and then winding the primary helix into a secondary form which forms a generally spherical shape, by winding the primary coil about poles placed on winding mandrel. The secondary wound coil is then annealed on the winding mandrel, and the coil is then removed from the winding mandrel detachably attached to a delivery means (pusher members) and loaded into a carrier for introduction into a delivery catheter. These widely used vaso-occlusive devices are generally constructed from a primary coil that is wound from a single wire of uniform material and most commonly is a Platinum alloy wire.

For some procedures, namely neurologic, the position of the embolic coil will be manipulated within the lesion by the physician. Therefore, the delivery systems require the coil to be attached to a disposable pusher and subsequently detachable upon activation. Prior to detachment of the coil, the physician may manipulate the position of the coil within a target site i.e. an aneurysm, by deploying and retracting it until an optimal position is obtained. Since the coils are generally made from Platinum, which is generally soft, there is a great risk that the coils may be damaged by unravelling if a significant tensile load is applied to them i.e. and in particular if the coil is entangled within a mass of other coils. Some detachable coils therefore are provided with means to limit the linear elongation of the coil, and is commonly referred to as 'stretch resistant'.

In another publication US patent US 5,582,619, an implantable vaso-occlusive device provided in the form of a vaso-occlusive coil and comprised of a primary helically wound coil which is then wound into a secondary shape. A stretch-resisting member is fixedly attached at both ends of the vaso-occlusive coil. These attachment points of the stretch-resisting member to the coil generally form rigid sections in the coil, particularly at the distal and proximal ends. These rigid points can adversely affect the flexibility of coil that can lead to disastrous clinical consequences such as perforation of the lesion being treated. It is therefore desirous to reduce or eliminate rigid sections on the coils, particularly at the distal end.

In yet another publication, US 8,029,464 a vasoocclusive coil is disclosed in which the coil is also reinforced with a stretch resistant member to improve safety during retraction of the coil. The stretch resistant member is fixedly attached at the distal end to the vaso-occlusive coil, and the other end of the stretch resistant member is detachably mounted to a detachment fibre that mounts the vaso-occlusive coil to the pusher member to allow for placement and release of the vaso-occlusive coil within the patient's vasculature. Such a configuration however is complicated and does not avoid the rigid attachment zone of the stretch resisting fibre to the distal end of the coil.

From the foregoing, it can be seen that prior stretch resisting means for vasoocclusive devices have provided important improvements in resisting elongation deformation during manipulations for the treatment of aneurysms and various types of arteriovenous anomalies, but there remains important limitations in the technology presently available.

It is an object of the present invention to provide a means for delivering a stretch resistant vaso-occlusive device that is improved both in regards to the handling by a physician during delivery to a target site and yet avoids the rigid attachment points that can compromise the flexibility of the coil and hence the safety of the device. It is another object of the present invention to provide a means for delivering a stretch resisting vaso-occlusive device that allows that the stretch resisting member extending through the lumen of the vaso-occlusive device can be easily removed after delivery to a target site. Moreover, it is an object of the present invention to provide a method of producing a system for delivering a stretch resisting vaso-occlusive device.

### Summary of the Invention

This object is achieved by the implant system of claim 1. With regards to the method, said object is achieved by the subject matter of claim 10.

The present invention provides a means for delivering a stretch resistant vasoocclusive device for occlusion of vascular anomalies comprising an outer helically wound primary coil having a proximal end and a distal end, defining a lumen between said proximal end and distal end, said proximal end of the vasoocclusive device is detachably mounted to the distal end of an elongated delivery means. The system further comprises an attachment element, in particular a first attachment element, proximate to the distal end of said vaso-occlusive device and a stretch resisting member extending through the lumen and through said attachment element wherein the stretch-resisting member has at least two proximal ends and at least one distal end and extending at least partially through said lumen and passing through said attachment element wherein said distal end of stretch resisting member is releasably attached to said distal attachment element. Furthermore, the attachment to the distal end of delivery means may be made by means of direct connection of the proximal end of the device and stretch resistant fibres, or by means of the attachment of the stretch resistant fibres only.

That is, the stretch resisting member extending through a lumen of the vasoocclusive device and around a distal attachment element has the advantages that it more flexible and that it provides the option that the stretch resisting member can be easily removed after delivery to a target site. Such an arrangement ensures that the flexibility of the vaso-occlusive device during manipulation within a target site is not impeded by the attachment zones of the stretch resisting member to the vaso-occlusive device. Moreover, providing a removable stretch resisting member provides additional benefits. The removal of the stretch resisting member after deployment may further improve the flexibility the vasoocclusive device or provide other features that are not the subject matter of this invention.

Furthermore, the costs associated with implanting the stretch resisting vasoocclusive device of the present invention to the health care organisations is reduced since the interconnection between the elongate delivery means and the vaso-occlusive device provides greater manipulation of the medical device at the target site, thus requiring less time by a physician, while additionally maintaining the crucial flexibility of the distal end of the device, thus potentially reducing adverse complications such as vessel perforations.

Further embodiments of the invention are indicated in the dependent claims.

In particular, at least one proximal end of the stretch resisting member may be detachably affixed to the distal end of the elongated delivery means. The at least one proximal end of the stretch resisting member is fixedly attached proximate to a proximal end of the vaso-occlusive device.The stretch resisting member may be formed of an elongated continuous loop of a fine element wherein the elongated loop is folded proximate at its mid-point forming the distal end and two opposing looped ends forming the proximal ends.

That is, the continuous loop of a fine element provides the advantage of forming a pair of looped ends that can be arranged in a variety of ways with the elongated delivery means and the vaso-occlusive device providing increased stretch resistance without compromising the flexibility of the vaso-occlusive device.

Further, the elongated delivery means may include a detachment segment that is disposed proximate to the two looped ends of the stretch resisting member. A second ring-like attachment element may be provided at the distal end of the elongated delivery means. Both ends of the stretch resisting member and the proximal end of the medical device may be attached to the second attachment element. Alternatively, one looped end of the stretch resisting member is fixedly attached to the elongated delivery means and the other end of the stretch resisting member is detachably affixed to the elongated delivery means thus providing for removal of the stretch resisting member after release of the vasoocclusive device.

The present invention also provides a method of producing a system for delivering a stretch resistant vaso-occlusive device including the steps of:
a. first forming an elongated closed loop of a fibrous element
b. folding the elongated closed loop proximate its midpoint forming a pair of first ends opposite a second end formed proximate the loop midpoint
c. attaching the pair of first ends to the distal end of a elongated delivery means
d. passing a second fibrous element through the second end of the loop
e. using the second fibrous element to pull the second end of the loop through the lumen of the vaso-occlusive device
f. attaching the second fibrous element to the distal end of the vasoocclusive device forming a releasable attachment junction

In a co-ordinate aspect of the present invention, a means for providing stretch resistance to an implantable device is provided wherein the device has a proximal end and a distal end, and has a length there between, and is comprised of a structure susceptible to adverse elongation when in traction, said device detachably mounted to the distal end of an elongated delivery means; and a stretch resisting member having proximal and distal ends extending at least partially along said length of said device, wherein said stretch resisting member is not fixedly attached to said device at any location along said length of said device, said stretch resisting member providing for enhanced resistance to elongation of said device prior to detachment from said delivery means.

The stretch resisting member is passed through an attachment element provided at a location along the length of said device, forming two proximal ends and one folded distal end, the said folded distal end of the stretch resisting member forming a flexible, releasable junction between said attachment element and stretch resisting member.

The stretch resisting member is detachably affixed to the elongated delivery means one of its proximal ends and affixed to the elongated delivery means at the other one of its proximal ends. Further, the stretch resisting member has two proximal ends and one said end is detachably affixed to the elongated delivery means and one said end is affixed to the elongated delivery means.

Furthermore, in the means for providing stretch resistance to an implantable device, the device has a proximal end and a distal end, and has a length there between, and is comprised of a structure susceptible to adverse elongation when in traction, said device detachably mounted to the distal end of an elongated delivery means. The stretch resisting member has proximal and distal ends extending at least partially along said length of said device, wherein said stretch resisting member is detachably affixed to the implantable device and provides for enhanced resistance to elongation of said device prior to detachment from said delivery means, and fully releasable from said device after detachment.

### Brief description of the Drawings

A better understanding of the features and advantages of the present invention will be obtained by reference to the following detailed description that sets forth illustrative embodiments by way of example only, in which the principles of the invention are utilized, and the accompanying drawings of which:
Figure 1 shows a partial cross-sectional view of an embodiment of a vasoocclusive device comprising a stretch resisting member according to the present invention.
Figure 2 shows a side view of a stretch resisting member formed as a continuous loop according to the present invention.
Figure 3 shows a side view of the stretch resisting member of figure 2 folded at its mid-point according to the present invention.
Figure 4 shows a side view of a distal end of an elongated delivery means comprising a distal loop being passed through the proximal ends of folded stretch resisting member according to the present invention.
Figure 5 shows a side view of folded stretch resisting member being pulled at its mid-point through distal loop of the elongated delivery means according to the present invention.
Figure 6 shows a side view of the folded stretch resisting member being further pulled through the distal loop of the elongated delivery means according to the present invention.
Figure 7 shows a side view of the stretch resisting member being pulled through the lumen of the vaso-occlusive device according to the present invention.

### Detail Description of the Invention

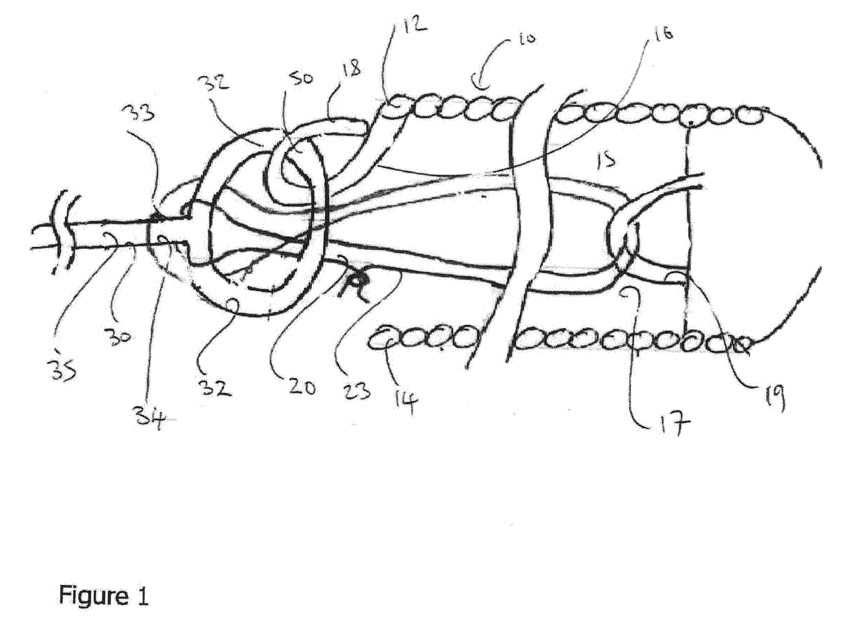

Figure 1 shows a simplified partial cross-sectional view of an embodiment of a vaso-occlusive device 10 that is implantable within a vasculature of a subject such as a patient. Vaso-occlusive device 10 may comprise an outer helically wound primary coil or helical winding 14. That is, vaso-occlusive device 10 may be generally defined as a flexible element that is formed from a primary winding composed of at least one wire 12 being helically wound to form a helical winding 14. Helical winding 14 may be composed of one or more wires (not shown) depending on for example the nature and location of the vascular anomaly. Generally, helical winding 14 is wound on a common longitudinal axis such as for example a mandrel (not shown) and made from, for example an implantable grade Pt-W alloy. As shown, internal diameter of helical winding 14 forms a longitudinal lumen 15. Vaso-occlusive device 10 further comprises a proximal end 16 and a distal end 17.

As shown in the current figure, proximal end 16 of helical winding 14 is provided with a loop segment 18 which is interconnected with loop 32 of elongated delivery means 30. That is, such an arrangement provides an engagement portion between elongated delivery means 30 and vaso-occlusive device 10 as will become apparent later. Furthermore, distal end 17 of vaso-occlusive device 10 is provided with an attachment element 19 that will be described in more detail later. Proximal end 16 of vaso-occlusive device 10 is preferably formed as a closed loop segment 18 so that it may interconnect with elongated delivery means 20 as will be described later. Alternatively, proximal end 16 may not include a closed loop segment 18 (not shown), wherein attachment of the vasoocclusive device 10 to the delivery means 30 is accomplished solely by attachment of the first ends stretch resisting member 20 to the distal end 33 of delivery means 30.

Further shown in the current figure is an elongated delivery means 30 which comprises a second attachment element, in particular a ring-shaped second attachment element, which in the embodiment shown is a loop 32 arranged at a distal end 33 of delivery means 30. Elongated delivery means 30 further comprises a detachment segment or zone 34 arranged on a core 35 of elongated delivery means 30 and further arranged proximal to loop 32. The purpose of detachment zone 34 is to allow separation of vaso-occlusive device 10 from elongated delivery means 30 once a physician has manipulated vaso-occlusive device 10 to a target site such as an aneurysm in the vasculature of a subject, such as a patient.

Detachment zone 34 may be a sacrificial element which is sacrificed when power is applied to the elongated delivery means 30 such that power is transferred across the exposed sacrificial segment 26 to electrolytically degrade segment the detachment zone 34 after the occlusion of an aneurysm.

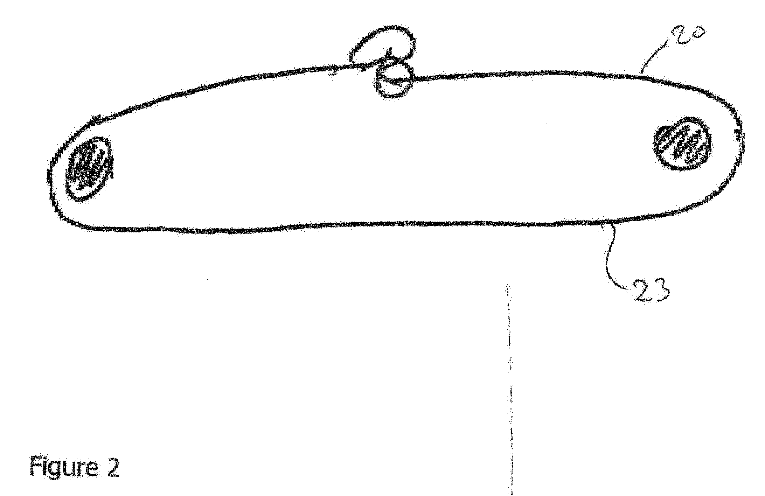

Shown disposed, and extending through longitudinal lumen 15 of vaso-occlusive device 10 is a stretch resisting member 20. Stretch resisting member 20 is formed of an elongated continuous loop 23 as seen more clearly in figure 2. Stretch resisting member 20, extending through lumen 15 of vaso-occlusive device 10, is affixed through attachment element 19 at the distal end 17 of vaso-occlusive device 10 as will be described later. Stretch resisting member 20 may be made from a fibre, in particular a fine polymer fibre, for example, 8-0 polypropylene or nylon suture material. Elongated continuous loop 23 may generally be formed from a fibrous element with a length of slightly less than four times the length of vaso-occlusive device 10. The continuous loop 23 may be formed by either gluing, heat fusing, or tying the ends of the length of stretch resisting member 20. Several stretch resisting members may be utilised for decreasing the likelihood of stretching of the vaso-occlusive device 10.

Further, stretch resisting member 20 is further affixed to the distal end of elongated delivery means 30. Loop 32 of elongated delivery means 30 is interconnected with loop segment 18 at proximal end 16 of helical winding 14. Such an arrangement provides an articulating junction 50 that allows the physician to easily manipulate vaso-occlusive device 10 by means of elongated delivery means 30 to a target site of a vasculature of a patient prior to the separation of the vaso-occlusive device 10 from the elongated delivery means 30. As is clearly depicted in the current figure however, articulating junction 50 composed of interconnected loops 18 and 32 is arranged at a position spaced from detachment zone 34 of elongated delivery device 30. That is, loop 32 is integrally formed from the distal end 33 of elongated delivery means 30 and engaged with attachment loop 18 at the proximal end 16 of vaso-occlusive device 10.

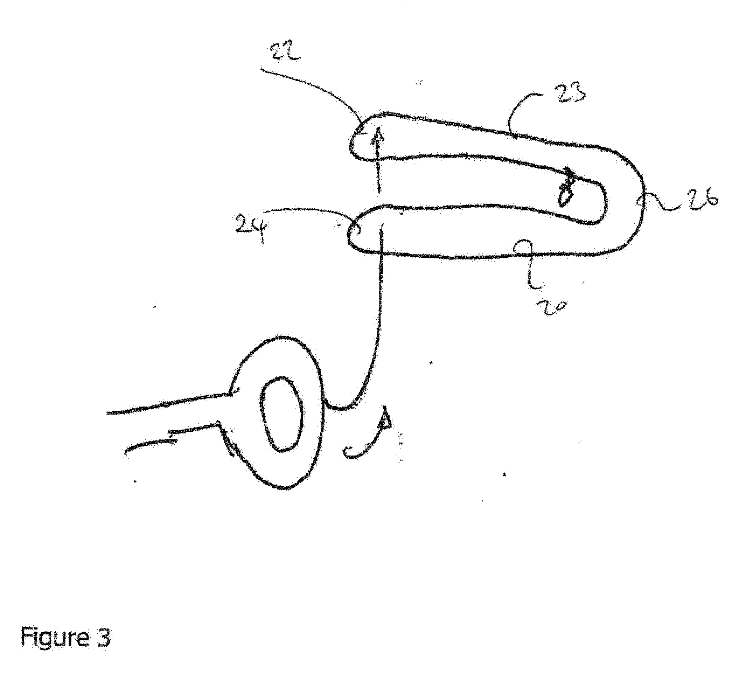

Figure 3 shows the elongated continuous loop 23 of the stretch resisting member 20 folded at its mid-point. Such a configuration provides a first proximal end 22 and a second proximal end 24, and further provides a distal end 26. Generally, the pair of proximal ends 22, 24 are attached to the delivery means 30, in particular a transport wire or guide wire.

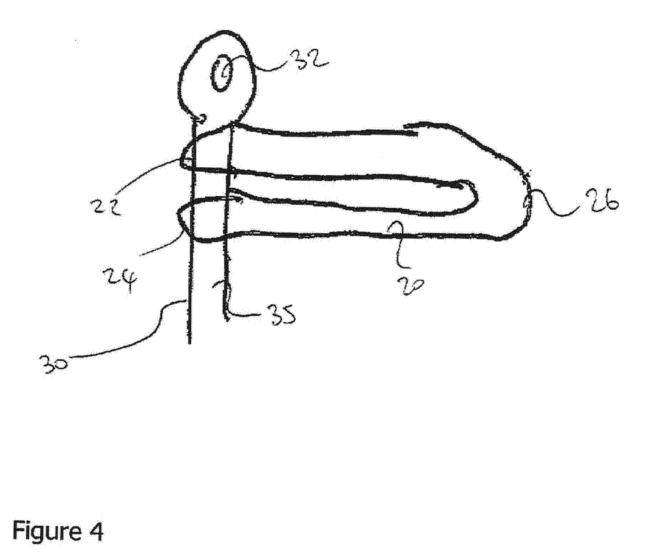

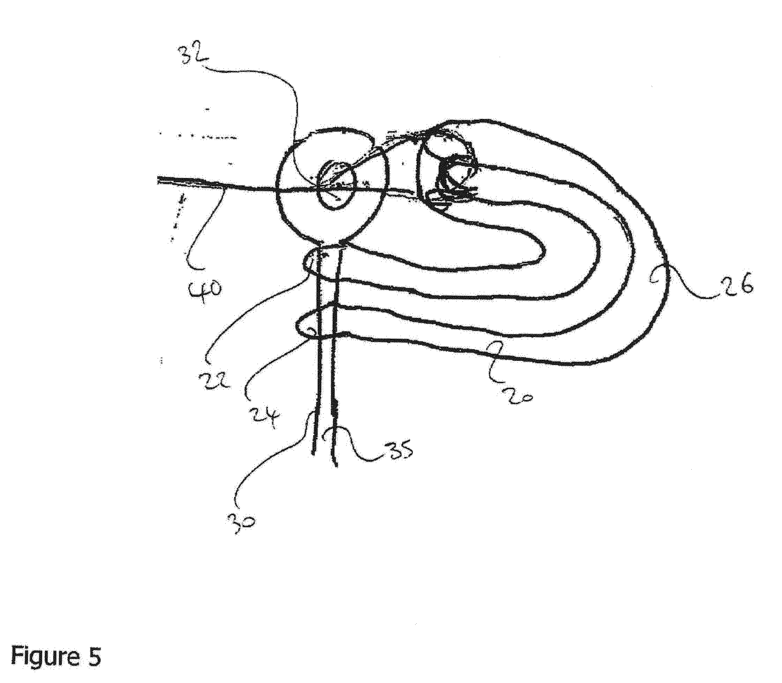

As shown in figure 4, first proximal end 22 and a second proximal end 24 are arranged to be looped through core 35 of elongated delivery means 30 such that distal end 26, that is the mid-point, of stretch resisting member 20 is free. That is, free distal end 26 shown in figure 5, and by means of an element 40, is pulled through loop 32 of elongated delivery means 30. That is, element 40 is interconnected to distal end 26 of stretch resisting member 20. In such a configuration, element 40 allows stretch resisting member 20 to be pulled through loop 32 of elongated delivery means 30. Element 40 may be 6-0 polypropylene or nylon suture material and selected to avoid damage to helical winding 14 and/or stretch resisting member 20.

In another configuration however (not shown), stretch resisting member 20 is looped at approximately at its mid-point around core 35 of elongated delivery means 30 with first proximal end 22 and second proximal 24 passed through loop 32 of elongated delivery means 30.

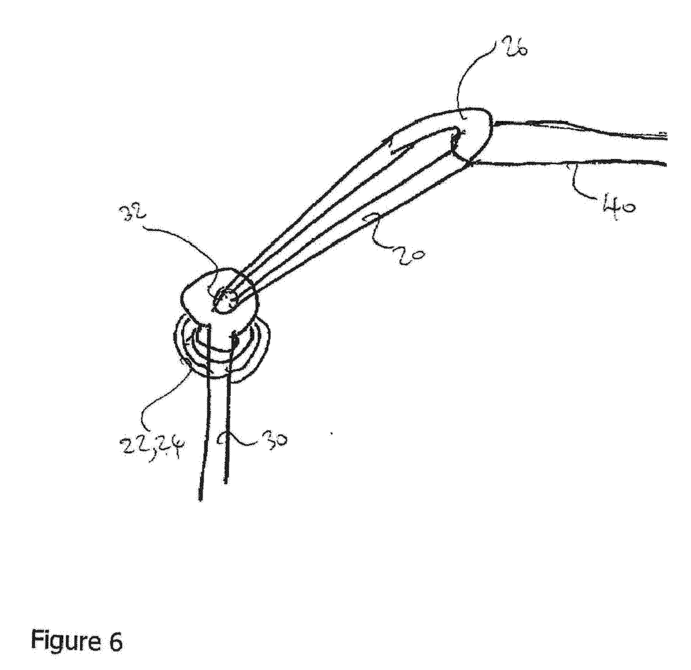

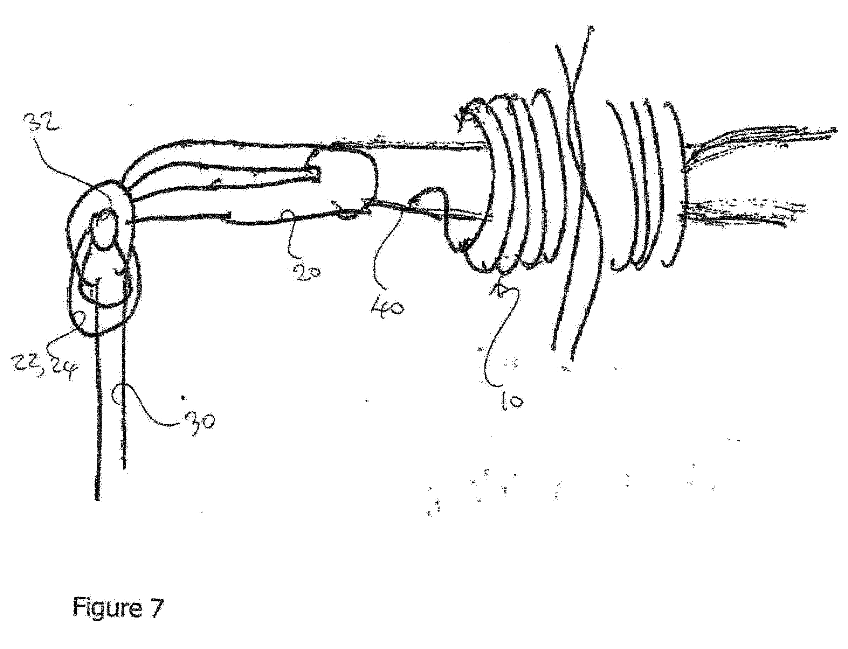

Figure 6 depicts a side view of folded stretch resisting member 20 being pulled at its mid-point through loop 32 of the elongated delivery means 30 such that any excess slack of stretch resisting member 20 is removed. In particular, element 40 is arranged so that it may further pull stretch resisting member 20 through lumen 15 of helical winding 14 as is shown in figure 7. The folded length of stretch resisting member 20 is configured such that the second end 26 will terminate proximate to, yet not extend past, the distal end 17 of vaso-occlusive device 10. Element 40 is then pulled tight though lumen 15 and formed into attachment element 19 (more clearly shown in Figure 1) that is subsequently formed via heat or gluing to integrally form the attachment element 19 and a hemispherical tip at distal end 17 thus forming an articulation junction between the attachment element 19 and the stretch resisting member 20.

In such an arrangement of a stretch resisting member 20 being looped around core 35 proximate to detachment zone 34 of elongated delivery means 30, when the vaso-occlusive device 10 is separated from the elongated delivery means 30, both the first proximal end 22 and a second proximal end 24 are also released from elongated delivery means 30.

In another alternate arrangement not shown, the first proximal end 22 of stretch resisting member 20 is looped around core 35 proximate to detachment zone 34 of elongated delivery means 30 and the second proximal end 24 of stretch resisting member 20 is fixedly attached to the distal end of elongated delivery means 30. When the vaso-occlusive device 10 is separated from the elongated delivery means 30 by means of electrolytic degradation of the detachment zone 34, the first proximal end 22 and vaso-occlusive device 10 are released and second proximal end 24 are also released from elongated delivery means 30. Moreover, since stretch resisting member 20 is fixedly attached at one end to the distal end of elongated delivery means 30 and passes through attachment element 19 such as a loop provided at distal end of vaso-occlusive device 10, it may be withdrawn from vaso-occlusive device 10 after the physician has manipulated vaso-occlusive device 10 to a target site in the vasculature of a subject and removes the elongated delivery means 30 thereby retracting the stretch resisting member 20. That is, one end, in particular the first proximal end 26, of the stretch resisting member 20 is fixedly attached to the elongated delivery means 30 and the other end, in particular the second proximal end 24, of the stretch resisting member 20 is detachably affixed to elongated delivery means 30.

The vaso-occlusive device 10 and elongated delivery means 30 are introduced through a delivery catheter means not shown, which is previously navigated through an insertion site of the subject, for example into the femoral artery near the groin, and through the vasculature to the target lesion site by the physician. By means of elongated delivery means 30 the physician may manipulate vasoocclusive device 10 into an optimal position within the target vascular lesion (not shown). Generally however, the physician would be guided to manipulate vasoocclusive device 10 by means of x-rays, CT scans and/or MRI scans to ensure positioning within the target location is correct.

Further vaso-occlusive device 10 may be detached from elongated delivery means 30 by means for example of electrolytic degradation at detachment zone 34, allowing a physician to remove elongated delivery means 30 and optionally the stretch resisting member 20 from the subject. That is, such removal of stretch resisting member 20 may provide for enhanced properties of the vaso-occlusive device 10 such as increased flexibility or other features that are not the subject matter of this invention.

In another example embodiment of the present invention, a length of stretch resisting member 20 and generally be a length of at least four times the length of vaso-occlusive device 10. The stretch resisting member 20 may be looped around core 35 of elongated delivery means 30 at approximately a mid-point of stretch resisting member 20 to provide a first free end and a second free end (not shown). Both free ends of the stretch resisting member 20 are passed through loop 32 of elongated delivery means 30 arranged at its distal end 33 and tightened as to form a tight connection.

Vaso-occlusive device 10 having helical winding 14 is then attached to elongated delivery means 30 by means of bending at least one of the windings at proximal end 16 of vaso-occlusive device 10 to form an angle which allows passing through loop 32 of elongated delivery means 30. The proximal end portion that is passed through loop 32 is then bent in such a way as to create a loop-in-loop junction between the elongated delivery means 30 and vaso-occlusive device 10.

The stretch resisting member 20 is then folded in two and pulled through the lumen 15 of the vaso-occlusive device 10 by means of a suitable device such as, for example a wire hook. The stretch resisting member 20 subsequently has two ends proximally, one set of ends looped around and attached to the elongated delivery means 30 and the other set are currently free ends and are kept from entangling.

Once the folded loop of stretch resisting member 20 is pulled through and is exterior to the distal end 17 of the vaso-occlusive device 10, a segment of a larger polymer fiber such as an end element are passed through the distal loops of the stretch resisting member 20. The stretch resisting member 20 is then retracted slightly into lumen 15 of vaso-occlusive device 10 (by a distance of just a few millimeters) and the end element is folded e.g. in half, to form a loop around the distal free ends of the stretch resisting member 20. Heat or glue is then applied to form the distal end 17 of the vaso-occlusive device 10 while also creating an internal distal hook for the stretch resisting member 20.

The free ends of the stretch resisting member 20 provided at the proximal end 16 of the vaso-occlusive device 10 would then be passed though loop 32 of elongated delivery means 30, and further looped around core 35 of elongated delivery means 30 (preferably in an opposite direction to the existing first loop) around core 35 and tightened to remove all slack within vaso-occlusive device 10. The free ends are then tied, bonded or melted together at a convenient location at the proximal end 16 of vaso-occlusive device 10. Again, as in the previous embodiment, since both ends of the stretch resisting member 20 are looped around the elongated delivery means 30 at the detachment zone 34, when vasoocclusive device 10 coils is detached, both ends of the stretch resisting member 20 will also be released. Since stretch resisting member 20 pass through a loop at the distal end of vaso-occlusive device 10, they are not fixedly attached and could be withdrawn from the coil after detachment.

In all the described embodiments herein, such stretch resisting configurations protect the vaso-occlusive device 10 from damage from tensile forces applied during manipulation by a physician and yet provide for proximal an distal flexible attachment means between the occlusive device 10 and stretch resisting member 20 thus ensuring that the risk of injury to a subject is reduced during a medical procedure.

## Claims

1. A system for delivering a stretch resistant vaso-occlusive device (10) wherein the vaso-occlusive device (10) comprises an outer helically wound primary coil (14) having a proximal end (16) and a distal end (17), defining a lumen (15) between said proximal end (16) and distal end (17), said vaso-occlusive device (10) detachably mounted to the distal end (33) of an elongated delivery means (30); and
- an attachment element (19), in particular a first attachment element, proximate to the distal end (17) of said vaso-occlusive device (10); and
- a stretch resisting member (20) having at least two proximal ends (22, 24) and at least one distal end (26) and extending at least partially through said lumen (15) and passing through said attachment element (19) wherein said distal end of stretch resisting member (20) is releasably attached to said distal attachment element (19).

2. The system of claim 1 wherein at least one proximal end of the stretch resisting member (20) is detachably affixed to the distal end (33) of the elongated delivery means (30).

3. The system of claim 1 wherein at least one proximal end of the stretch resisting member (20) is fixedly attached proximate to the proximal end (12) of the vaso-occlusive device (10).

4. A system of any of claim 1 to 3 wherein said stretch resisting member (20) is formed of a elongated continuous loop (23) of a fine element wherein the elongated loop (23) is folded proximate its mid-point forming the distal end (26) and the two opposing looped ends forming the proximal ends (22, 24).

5. A system of any of claims 1 to 4 wherein said elongated delivery means (30) includes a detachment zone (34) that is disposed proximate to at least one of the two proximal ends of the stretch resisting member (20).

6. A system of any of claims 1 - 5 wherein a second ring-like attachment element (32) is provided at the distal end (33) of the elongated delivery means (30).

7. The system of claim 6 wherein at least one proximal end of the stretch resisting member (20) is attached to the second attachment element (32).

8. The system of claim 6 wherein at least one proximal end of the stretch resisting member (20) and the proximal end (16) of the vaso-occlusive device (10) are attached to the second attachment element (32).

9. A system of any of claims 1- 8 wherein one proximal end (22) of the stretch resisting member (20) is fixedly attached to the elongated delivery means (30) and the other proximal end (24) of the stretch resisting member (20) is detachably affixed to the elongated delivery means (30).

10. A method of producing a system for delivering a stretch resistant vasoocclusive device (10) including the steps of:
a. first forming an elongated closed loop of a fibrous element
b. folding the elongated closed loop proximate its midpoint forming a pair of first ends (22, 24) opposite a second end (26) formed proximate the loop midpoint
c. attaching the pair of first ends (22, 24) to the distal end (33) of a elongated delivery means (30)
d. passing a second fibrous element (40) through the second end of the loop (26)
e. using the second fibrous element (40) to pull the second end (26) of the loop through the lumen (15) of the vaso-occlusive device (10)
f. attaching the second fibrous element (40) to the distal end (33) of the vaso-occlusive device (30) forming a releasable attachment junction.

11. A means for providing stretch resistance to an implantable device (10) wherein the device (10) has a proximal end (16) and a distal end (17), and has a length there between, and is comprised of a structure susceptible to adverse elongation when in traction, said device (10) detachably mounted to the distal end (33) of an elongated delivery means (30); and
a stretch resisting member (20) having proximal and distal ends extending at least partially along said length of said device, wherein said stretch resisting member (20) is not fixedly attached to said device (10) at any location along said length of said device (10), said stretch resisting member (20) providing for enhanced resistance to elongation of said device (10) prior to detachment from said delivery means (30).

12. The means for providing stretch resistance to an implantable device (10) according to claim 11, wherein the stretch resisting member (20) is passed through an attachment element (19) provided at a location along the length of said device (10), forming two proximal ends (22,24) and one folded distal end (26), the said folded distal end (26) of the stretch resisting member (20) forming a flexible, releasable junction between said attachment element (19) and stretch resisting member (20).

13. The means for providing stretch resistance to an implantable device (10) according to claim 12, wherein the stretch resisting member (20) is detachably affixed to the elongated delivery means (30) one of its proximal ends (22) and affixed to the elongated delivery means (30) at the other one of its proximal ends.

14. The means for providing stretch resistance to an implantable device (10) according to claim 12, wherein the stretch resisting member (20) has two proximal ends (22, 24) and one said end is detachably affixed to the elongated delivery means (30) and one said end is affixed to the elongated delivery means (30).

15. A means for providing stretch resistance to an implantable device (10) wherein the device (10) has a proximal end (16) and a distal end (17), and has a length there between, and is comprised of a structure susceptible to adverse elongation when in traction, said device (10) detachably mounted to the distal end (33) of an elongated delivery means (30); and
a stretch resisting member (20) having proximal and distal ends (22, 24) extending at least partially along said length of said device, wherein said stretch resisting member (20) is detachably affixed to the implantable device (10) and provides for enhanced resistance to elongation of said device (10) prior to detachment from said delivery means (30), and fully releasable from said device (10) after detachment.
